Europäisches Patentamt

**European Patent Office**

**Office européen des brevets**

(19)

(11) Publication number: **0 145 334**
A2

(12) **EUROPEAN PATENT APPLICATION**

(21) Application number: **84307953.4**

(22) Date of filing: **16.11.84**

(51) Int. Cl.⁴: **C 07 C 47/55, C 07 C 45/00**

(30) Priority: **22.11.83 GB 8331120**

(71) Applicant: **Pfizer Limited, Ramsgate Road, Sandwich Kent CT13 9NJ (GB)**
(84) Designated Contracting States: **GB**

(71) Applicant: **Pfizer Corporation, Calle 15 1/2 Avenida Santa Isabel, Colon (PA)**
(84) Designated Contracting States: **BE DE FR IT LU NL**

(43) Date of publication of application: **19.06.85 Bulletin 85/25**

(72) Inventor: **Cross, Peter Edward, 21 Cherry Avenue, Canterbury Kent (GB)**
Inventor: **Thomas, Geoffrey Noel, 33 Swaynes Way Eastry, Nr. Sandwich Kent (GB)**

(84) Designated Contracting States: **BE DE FR GB IT LU NL**

(74) Representative: **Wood, David John et al, Pfizer Limited Ramsgate Road, Sandwich Kent CT13 9NJ (GB)**

(54) **Benzaldehyde derivative and process for its preparation.**

(57) 2-Chloro-3-trifluoromethylbenzaldehyde and a process for preparing it characterised by (i) reacting 2-chloro-1-trifluoromethylbenzene with a $C_1$–$C_4$ alkyl lithium or phenyl lithium in an organic solvent at − 60 °C or below (ii) reacting the product of step (i) with dimethylformamide at − 60 °C or below and (iii) reacting the product of step (ii) with water.

2-Chloro-3-trifluoromethylbenzaldehyde is especially useful as an intermediate in the synthesis of 4-(2-chloro-3-trifluoromethylphenyl)-1,4-dihydropyridine calcium antagonists, especially 2-(2-aminoethoxymethyl)-4-(2-chloro-3-trifluoromethylphenyl)-3-ethoxycarbonyl-5-methoxycarbonyl-6-methyl-1,4-dihydropyridine.

This invention relates to 2-chloro-3-trifluoromethylbenzaldehyde and to a process for making it.

The process for preparing 2-chloro-3-trifluoromethylbenzaldehyde is characterised by the following steps:-

(i) reacting 2-chloro-trifluoromethylbenzene with a $C_1$-$C_4$ alkyl lithium or phenyl lithium, the reaction being carried out in an organic solvent at -60°C or below;

(ii) reacting the product of step (i) with N,N-dimethylformamide (DMF) at -60°C or below;

and (iii) reacting the product of step (ii) with water.

Preferred lithiums for use in step (i) are phenyl, methyl, n-butyl, sec.-butyl and t-butyl lithium. It is most convenient to use n-butyl lithium. The preferred organic solvent is dry tetrahydrofuran (THF). Step (ii) is preferably carried out by adding DMF dropwise as a solution in an organic solvent such as dry THF. Steps (i) and (ii) must be carried out at low temperature (-60°C or below and preferably -60° to -80°C). Step (iii), the reaction with water, is typically carried out at 5-25°C. Step (iii) is most conveniently carried out at room temperature. The product can be isolated and purified conventionally, typically by solvent extraction followed by purification of the crude aldehyde product with sodium bisulphite in a conventional manner. A typical isolation and purification procedure is described in the Example hereinafter.

0145334

2-Chloro-3-trifluoromethylbenzaldehyde is especially useful as an intermediate in the preparation of certain pharmaceuticals, namely 4-(2-chloro-3-trifluoromethylphenyl)-1,4-dihydropyridines which are active calcium antagonists, especially 2-(2-amino-ethoxymethyl)-4-(2-chloro-3-trifluoromethylphenyl)-3-ethoxycarbonyl-5-methoxycarbonyl-6-methyl-1,4-dihydropyridine.

The above process provides a simple, cheap and safe route to 2-chloro-3-trifluoromethylbenzaldehyde in useful yield.

The invention is illustrated by the following Example, in which all temperatures are in °C:-

## EXAMPLE

### Preparation of 2-chloro-3-trifluoromethylbenzaldehyde

2-Chloro-1-trifluoromethylbenzene (54.15 g) was dissolved in dry tetrahydrofuran (500 ml) and stirred while cooling to -68° under a stream of dry nitrogen. (The whole reaction is carried out under dry nitrogen until the addition of distilled water.) To this was added n-butyl lithium (180 ml of a 1.6 M solution in hexane) dropwise keeping the temperature below -60°. After stirring at -68° for a further 2 hours, a solution of dimethylformamide (22 ml) in dry tetrahydrofuran (100 ml) was added dropwise keeping the temperature below -60°. The reaction mixture was allowed to warm to room temperature slowly over 17 hours and distilled water (200 ml) was then added. The organic phase was separated off and the aqueous liquors were extracted with ether (100 ml). The combined ether extracts plus the organic phase were washed with saturated brine, dried ($MgSO_4$), filtered and evaporated to give 61.5 g of an orange oil, being the crude title compound.

This oil was then added to an aqueous sodium bisulphite solution (65 g in 600 ml distilled water) and heated at 60° for 0.5 hours. The solution was extracted with methylene chloride (3 x 100 ml) and, after acidification of the aqueous phase with concentrated sulphuric acid to pH 1.0, was heated at 100° for a further 0.5 hours. The resultant aqueous solution was extracted with methylene chloride (3 x 200 ml) and the combined organic extracts were dried ($MgSO_4$), filtered and evaporated to give 42 g of a colourless solid which was crystallised from hexane to give the title compound, m.p. 43-44°.

PLC 388

## CLAIMS:

1.  2-Chloro-3-trifluoromethylbenzaldehyde.

2.  A process for preparing 2-chloro-3-trifluoromethyl-benzaldehyde, characterised by the following steps:-

(i)  reacting 2-chloro-1-trifluoromethylbenzene with a $C_1$-$C_4$ alkyl lithium or phenyl lithium, the reaction being carried out in an organic solvent at -60°C or below;

(ii)  reacting the product of step (i) with N,N-dimethylformamide (DMF) at -60°C or below; and (iii) reacting the product of step (ii) with water.

3.  A process according to claim 2, characterised in that n-butyl lithium is used in step (i).

4.  A process according to claim 2 or 3, characterised in that the organic solvent used in step (i) is tetrahydrofuran.

5.  A process according to claim 4, characterised in that in step (ii) the dimethylformamide is added as a solution in tetrahydrofuran.

6.  A process according to any one of the preceding claims, characterised in that steps (i) and (ii) are carried out at temperatures of from -60° to -80°C.

7.  A process according to any one of claims 2 to 6 characterised in that step (iii) is carried out at a temperature of from 5 to 25°C.